# EUROPEAN PATENT APPLICATION

(11) **EP 2 269 510 A2**
(43) Date of publication of application: **05.01.2011**
(21) Application number: 10183150.1
(22) Date of filing: 24.02.2003
(51) Int. Cl.: A61B 5/15

(54) **Minimum invasive optical format with integrated lance**

(30) Priority: 05.03.2002 US 361401 P
(62) Divisional of application: 03003247.8
(71) Applicant: Bayer Healthcare LLC, Tarrytown, NY 10591-5097 (US)
(72) Inventor: Dosmann, Andrew J., GRANGER, INDIANA 46530, IN (US)
(74) Representative: Linhart, Angela

(57) **Abstract**

A disposable optical format for lancing the skin of a patient and harvesting blood to determine blood chemistries such as glucose level includes a housing with openings defining an optical path. A translucent hollow capillary tube with multiple planar sides and an end cleaved to a sharp edge is mounted in the housing. The sides of the tube are formed of an optical material such as fused silica. Significantly less pain, high probability of blood harvesting and improved overall test time are achieved with integrating the lance, harvest and analysis operation.

## Description

### FIELD OF THE INVENTION

The present invention relates to minimum invasive techniques to determine compositions of body fluids. More particularly, the present invention relates to an optical format with a square fused silica lance for piercing skin of a user to harvest blood for testing.

### BACKGROUND OF THE INVENTION

Current methods of monitoring components of body fluids such as blood glucose involve painful skin punctures using steel needles having diameters from 28 (360 um) to 24 (550 um) gauge. Approximately thirty percent of steel lance skin punctures do not produce a blood sample thus requiring repeated puncturing. This increases the pain experienced by a patient causing some patients to avoid or skip testing. In order for prior art to conduct a proper analysis of a body fluid sample, a patient must lance and then manually harvest a minimum of 300 nL of body fluid such as blood into a format or a strip. An electrochemical or optical analysis of a chemical reaction is then performed to determine the levels of the desired component.

### SUMMARY OF THE INVENTION

The present invention is directed to a lance and optical format that can puncture the skin of a patient with little resulting pain, significantly improve reliability of blood production, automatically harvest a small blood sample, and analyze the sample with conventional transmission spectrometry. The lance is defined by a square, fused silica capillary tube. One end of the tube is cleaved to a sharp point that serves to pierce the skin of a patient. The other end of the lance is secured in a housing that has windows or aligned openings defining an optical path.

A blood sample is produced by first piercing a patient's skin followed by either vacuum or mechanical pressure around the wound to enhance blood flow. The lance is returned to a drop of blood at the puncture and the sample is harvested. Capillary action draws the sample into the lance. The sample reacts with reagents coated onto the walls inside the capillary, which produces a color change to the sample. Optical analysis of the sample can be performed using transmission spectrometry by passing a beam of light through the lance to a detector.

### BRIEF DESCRIPTION OF THE FIGURES

Other objects and advantages of the invention will become apparent upon reading the following detailed description and upon reference to the drawings in which:

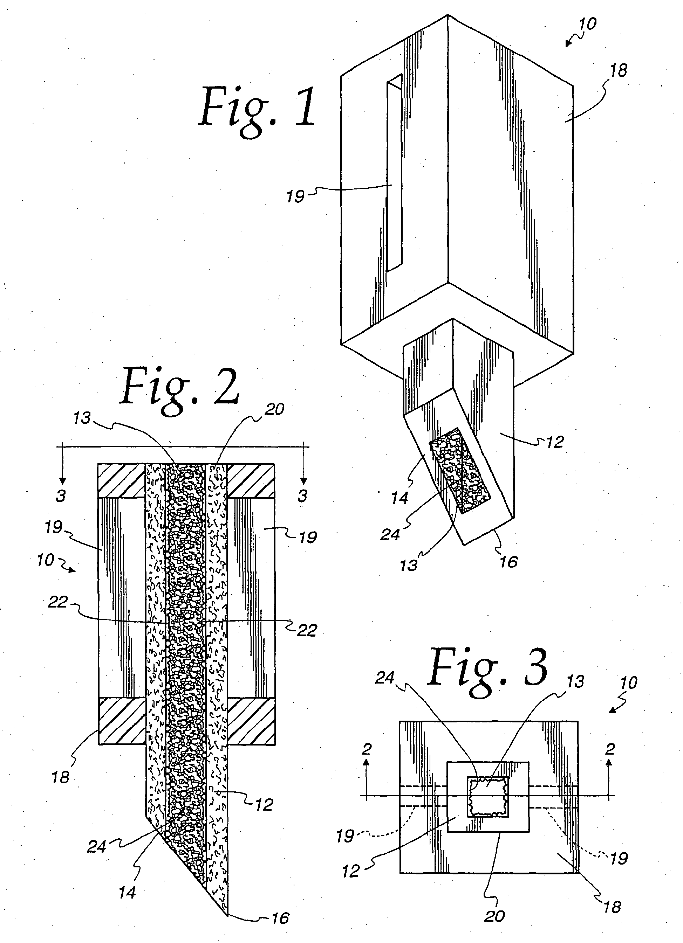

FIG. 1 is a perspective view of a minimum invasive optical format with an integrated lance constructed in accordance with the principles of the present invention;
FIG. 2 is a cross sectional view of the minimum invasive optical format with an integrated lance taken along line 2-2 of FIG. 3; and
FIG. 3 is a top plan view of the minimum invasive optical format with an integrated lance taken along line 3-3 of FIG. 2.

While the invention is susceptible to various modifications and alternative forms, a specific embodiment thereof has been shown by way of example in the drawings and will herein be described in detail. It should be understood, however, that it is not intended to limit the invention to the particular forms disclosed, but on the contrary, the intention is to cover all modifications, equivalents, and alternatives falling within the spirit and scope of the invention as defined by the appended claims.

### DETAILED DESCRIPTION OF THE ILLUSTRATED EMBODIMENTS

Referring to the drawings, a minimum invasive optical format with an integrated lance generally designated by the reference numeral 10 is illustrated. The format with integrated lance 10 includes a lance 12 formed from a square fused silica capillary tubing manufactured by Polymicro Technologies of Phoenix, Arizona. The lance 12 can have other shapes such as rectangular depending on the intended use of the format with integrated lance 10.

The lance 12 is hollow and has a square channel 13 which can have a width of 50 um, 75 um or 100 um and a length of 5 mm. These dimensions correspond to volumes of 13 nl, 29 nl, and 50 nl, respectively. A chemistry or reagent indicator 22 is dried onto an inside wall 24 of the channel 13. The chemistry 22 has an indicator formulation that is sensitive to an analyte being read. For example, if the analyte is glucose, the chemistry could be reductive hexokinase or glucose dehydrogenase. A first end 14 of the lance 12 is cleaved to a sharp point 16 at an angle of 45° ± 15° that serves to pierce the skin of a patient. The sharp point 16 at an angle of 45° ± 15° aids in cleanly cutting the skin as well as blood capillaries below the skin. Cutting blood capillaries improves the reliability of producing a blood sample to 98%.

To minimize pain when skin is pierced by the sharp point 16 of the lance 12, it is preferred that the outside dimension of the lance 12 be small. By using a square fused silica tube for the lance 12, the outside dimension of the lance 12 is 300 um which is smaller than a similar dimension of a typical 28 gauge steel lance which has a diameter of 360 um. Lance 10 can be modified depending on the intended use.

The format with integrated lance 10 also includes a housing 18 mounted on a second end 20 of the lance 12. The housing 18 has opposed windows or openings 19. The housing 18 controls the depth of a puncture into a patient's skin by the lance 12. The depth of a puncture corresponds to the length of the lance 12 extending out of the housing 18. In one embodiment, the portion of the lance 12 extending outside of the housing 18 measures 2 mm. The housing 18 also provides support for the lance 12 and resists breakage of the lance 12. Breakage of the lance 12, however, is minimized due to the strength of fused silica which is based on a Si-O bond which has a theoretical tensile strength of 2000 kpsi.

The optical format with integrated lance 10 is used to pierce the skin of a patient with the sharp point 16. Once a drop of blood appears at the puncture site, the tip or first end 14 of the lance 12 is returned to the drop of blood and capillary action draws a sample into the square channel 13. Another embodiment would leave the lance below the surface of the skin until capillary action or vacuum assisted capillary action obtains a sample. The sample is allowed to react with the dried chemistry 22 coated onto the capillary walls which produces a color change to the sample. The change in color is proportional to analyte concentration. The sample is then read with transmission spectrometry by passing a monochromatic collimated beam of light through the portion of the lance 12 in the housing 18 by passing the beam through one of the openings 19 in the housing 18. The opening 19 can be used to mask the beam down to only the sample area in the lance 12. A detector is located adjacent the other opening 19. The flat surfaces of the lance 12 provide an excellent optical window and the optical transmission of fused silica is spectrally flat from UV into infrared. Thus, readings can be done at several wavelengths to correct for interferences. For example, hematocrit levels in whole blood can interfere with glucose concentration determination. Hamatocrit could be determined at a wavelength that is independent of glucose. Glucose concentration can then be corrected. In addition, the square shape of the capillary 12 provides a two times increase in transverse optical interaction path length compared to round capillaries. The square shape of the lance 12 also provides alignment between the openings 19 in the housing 18 and the sample in the lance 12.

While the present invention has been described with reference to one or more particular embodiments, those skilled in the art will recognize that many changes may be made thereto without departing from the spirit and scope of the present invention. Each of these embodiments and obvious variations thereof is contemplated as falling within the spirit and scope of the claimed invention, which is set forth in the following claims.

## Claims

1. A method for analyzing a sample of body fluid, comprising:
drawing a sample of body fluid into a channel of a lance, the sample reacting with a reagent on an inside wall of the channel at a sample area;
passing light through a window of the lance and to the sample area, the lance including a multisided tube formed from fused silica, the window being formed by a planar surface of the multisided tube;
detecting the light from the sample area; and
determining, from the detected light, a composition of the sample.

2. The method of claim 1, wherein passing the light through the window includes passing a monochromatic collimated beam of light through the window to the sample area.

3. The method of claim 1, wherein detecting the light from the sample area includes detecting a change in color when the sample reacts with the reagent.

4. The method of claim 1, wherein passing the light through the window includes passing light of several wavelengths through the window to the sample area.

5. The method of claim 4, further comprising determining an interference based on detecting the light of several wavelengths and correcting the composition of the sample for the interference.

6. The method of claim 5, wherein determining the composition of the sample includes determining a glucose concentration for a blood sample, passing light of several wavelengths includes passing a wavelength that is independent of glucose to determine a hematocrit level of the blood sample, and determining a composition of the sample includes correcting the glucose concentration for an interference associated with the hematocrit level.

7. The method of claim 1, wherein detecting the light from the reaction includes detecting the light through another window of the lance.

8. The method of claim 1, wherein a housing is mounted around a portion of the lance and includes an opening, and passing the light through the window includes passing the light through the opening of the housing to the window.

9. The method of claim 8, wherein the opening provides a mask to beam the light to the sample area.

10. The method of claim 8, wherein the housing includes two opposing openings, passing the light through the window includes passing the light through one of the openings, and detecting the light from the reaction includes detecting the light through the other of the openings.

11. The method of claim 1, wherein the lance includes a rectangular shape.

12. The method of claim 1, wherein determining the composition of the sample includes determining a glucose concentration for a blood sample.
